# EUROPEAN PATENT SPECIFICATION

(11) **EP 1 567 191 B1**
(45) Date of publication and mention of the grant of the patent: **08.09.2010**
(21) Application number: 03789094.4
(22) Date of filing: 26.11.2003
(51) Int. Cl.: A61K 39/39

(54) **NOVEL AMINE-BASED ADJUVANT**
NEUE ADJUVANT AUF DER BASIS VON AMINEN
NOUVEL ADJUVANT A BASE D'AMINE

(30) Priority: 26.11.2002 DK 200201827
(43) Date of publication of application: 31.08.2005
(73) Proprietor: Eurocine Vaccines AB, 171 77 Stockholm (SE)
(72) Inventor: SCHRÖDER, Ulf, S-174 50 Sundbyberg (SE); HINKULA, Jorma, S-131 35 Nacka (SE)
(74) Representative: Johansen, Marianne
(86) International application number: PCT/EP2003/013348
(87) International publication number: WO 2004/047862

(56) References cited:
- WO-A-00/47224
- US-A- 4 323 561
- US-A- 4 855 283
- LINGNAU KAREN ET AL: "Poly-L-arginine synergizes with oligodeoxynucleotides containing CpG-motifs (CpG-ODN) for enhanced and prolonged immune responses and prevents the CpG-ODN-induced systemic release of pro-inflammatory cytokines." VACCINE, vol. 20, no. 29-30, 2002, pages 3498-3508, XP004381814 4 October, 2002 ISSN: 0264-410X cited in the application
- O'HAGAN D ET AL: "Induction of potent immune responses by cationic microparticles with adsorbed human immunodeficiency virus DNA vaccines." JOURNAL OF VIROLOGY. OCT 2001, vol. 75, no. 19, October 2001 (2001-10), pages 9037-9043, XP0002964854 ISSN: 0022-538X
- Proc. Natl. Acad. Sci. USA (2000) 97:811-616
- Vaccine (1997) 15:818-820
- Vaccine (2001) 19:1911-1923
- Vaccine (2002) 20:3389-3398
- Adv. Drug Delivery Rev. (2001) 51:81-96

## Description

### Field of the invention

The present invention relates to a novel adjuvant composition for administration of a polynucleotide or a polypeptide to an animal in order to enhance the immunological response against the polypeptide, or the polypeptide expressed as a result of the administration of the polynucleotide.

### Background of the invention

Within the last decade plasmids encoding antigens have revolutionized
vaccine design. Although no DNA vaccine has yet been approved for routine human or veterinary use, the potential of this vaccine modality has been demonstrated in experimental animal models.

Plasmid DNA vaccination has shown efficacy against viral, bacterial and parasitic infections modulated the effects of autoimmune and allergic diseases and induced control over cancer progression. Because of the simplicity and versatility of these vaccines, various routes and modes of delivery are possible to engage the desired immune responses. These may be T or B effector cell responses able to eliminate infectious agents or transformed cells. DNA vaccines may also induce an immunoregulatory/modulatory or immunosuppressive (tolerizing) response that interferes with the differentiation, expansion or effector functions of B and T cells. Pre-clinical and initial small-scale clinical trials have shown DNA vaccines in either of these modes to be safe and well tolerated. DNA vaccines have proven very effective in small animal models and are also effective in humans and larger animals, including primates, cattle, horses, and swine.

However, when DNA is administered alone, the administration of a relatively high amount of DNA (several milligrams) is required to give a long-lasting and strong immune response in humans and larger animals.

However, although the use of DNA vaccines at milligram doses is feasible, it would impose serious limitations on the number of constructs that could be included in a vaccine. In addition, the use of very high doses of DNA is less favorable from a process economic standpoint. Therefore, there is a clear need to induce effective immunity in humans with lower and fewer doses of DNA, as well as to increase the magnitude of the immune responses obtained.

A number of such systems that can evoke a protective immune response have been tested, and the majority of these rely on the use of cationic molecules that are co-administered with the DNA.

Substances that have been described in the literature include lactic acid particles with dioleyl-1,3-trimethylammonioproane (PNAS 2000, 97(2) 811), liposomes containing DMRIE/DOPE (Vaccine 1997, 15(8) 818), Vaxfectin liposomes (Vaccine 2001, 19, 1911-23) and poly-I-arginine combined with CpG motifs is described in Vaccine 20 (2002) 3498-3508.

Furthermore, cationic emulsions were made by the inclusion of either 1,2-dioleoyl-3-trimethyl-ammonium-propane (DOTAP) or the Th1 inducing adjuvant dimethyl, dioctadecyl ammonium bromide (DDA) (Vaccine 20 (2002) 3389-3398). A cationic polysaccharide, Chitosan, has also been described as a delivery system for DNA and poly-peptides (Advanced Drug Delivery Reviews 51 (2001) 81-96).

### Detailed description of the invention

As described above, one of the main problems of DNA-based vaccines is their poor generation of antibodies upon administration to humans. Thus, the present invention provides adjuvants that are able to increase and/or modulate the immune response of an organism to nucleic acid-based or peptide-based vaccines and/or antigens.

Furthermore, as compared to many of the known DNA adjuvants, the adjuvants according to the invention show a very high degree of stability, i.e. it is possible to store them at room temperature for more than 6 months.

The adjuvants according to the invention comprise one or more positively charged (cationic) substances. When the adjuvants are administered together with polynucleotides, which normally have a negative charge, or polypeptides, which may be either negatively or positively charged, there will be electrostatic interactions between the components. Such interactions are of vital importance in order to obtain an enhanced immune response.

Accordingly, the invention relates to an adjuvant for use in a vaccine, the adjuvant comprising one or more acyl amines comprising from 4 to 30 carbon atoms and mixtures thereof.

### Definitions

Throughout the text including the claims, the following terms shall be defined as indicated below.

The term "acyl" or "acyl group" encompasses natural or synthetic, branched or unbranched, cyclic or acyclic, substituted or unsubstituted acyl, alkyl, alkenyl and alkynyl chains of from 4 to 30 carbon atoms, such as, e.g., from 6 to 24 carbon atoms, from 8 to 20 carbon atoms or from 12 to 20 carbon atoms.

The term "acyl amine" encompasses acyl, alkyl, alkenyl or alkynyl chains onto which an amine group has been attached.

The term "antigen" is defined as anything that can serve as a target for an immune response. The immune response can be either cellular or humoral and be detected in systemic and/or mucosal compartments.

The term "vaccine" is defined herein as a suspension or solution or antigenic moieties, usually consisting of infectious agents, or some part of the infectious agents, that is introduced to an animal body to produce active immunity.

The term "adjuvant" as used herein is any substance whose admixture with an injected immunogen increases or otherwise modifies the immune response.

The term "gp41" means the gp41 transmembrane HIV envelope glycoprotein and any fragments or variants, alleles, analogs and derivatives thereof.

By the term "gp41 nucleotide" is intended to mean a nucleotide encoding the gp41 protein or fragments thereof.

The term "L3" as used herein is defined as an adjuvant for classic vaccines comprising a monoglyceride and a fatty acid as described in PCT/SE97/01003, published as WO/1997/047320.

The term "N3" as used herein is defined as the adjuvant formulation according to the present invention and thus is intended to mean an adjuvant comprising one or more acyl amines comprising from 4 to 30 carbon atoms.

In one embodiment of the invention the adjuvant may be one or more acyl amines, selected from e.g. a lauryl amine (C12), palmityl amine (C16), palmitoleyl amine (C16:1), oleyl amine (C18:1) and linoleyl amine (C18:2).

In a further embodiment of the invention the adjuvant may be a mixture of oleyl amine and lauryl amine. The w/w ratio of oleyl amine to lauryl amine may be from about 0.1 to about 10, such as, e.g., from about 0.25 to about 9, from about 0.5 to about 8, from about 0.75 to about 7, from about 1 to about 6, from about 1 to about 5, from about 1 to about 4, from about 1 to about 3, from about 1 to about 2 and from about 1 to about 1.

The adjuvant according to the invention may further comprise a monoglyceride having the formula 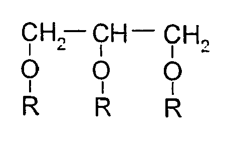 wherein R is selected from H and an acyl group containing from 6 to 30 carbon atoms with the proviso that two of the R groups are H. In a monoglyceride the acyl chains are normally placed on carbon atom 1 or 3 of the glycerol backbone, but there will often be a acyl migration between the carbon atoms 1 and 3, and the center carbon atom 2, resulting in that approximately 90% of the acyl chains will be positioned on the carbon atom 1 or 3, and about 10% will be positioned on the center carbon atom.

In the present invention is used distilled 1-monoglyceride from Danisco Ingredients (Denmark) with a purity of at least 80% w/w, such as, e.g., at least 90% w/w or at least 95% w/w. The content of diglyceride in the 1-monoglyceride is at the most 3% and the content of triglycerides and fatty acids are less than 1%.

In one embodiment of the invention, the adjuvant may be a mixture of a monoglycerides and an acyl amine, such as, e.g., mono-olein and oleyl amine.

The w/w ratio of mono-olein to oleyl amine may be from about 0.1 to about 10, such as, e.g., from about 0.25 to about 9, from about 0.5 to about 8, from about 0.5 to about 7, from about 0.5 to about 6, from about 1 to about 5, from about 1 to about 4, from about 1 to about 3, from about 1 to about 2 and from about 1 to about 1.

In a specific embodiment, the w/w ratio of mono-olein to oleyl amine may be 0.45.

The adjuvant according to the invention contains the adjuvant components, i.e. the cationic substances and optionally one or more monoglycerides in a concentration that elicits an immune response in a human or animal to an antigen administered to the human or animal.

One embodiment of the invention relates to an adjuvant dispersed in a medium. When the adjuvant is dispersed in a medium the term "adjuvant-containing medium" is used to describe the medium.

In an aspect of the invention, the medium may be in the form of an aqueous medium such as an aqueous suspension.

The medium may further comprise an immune stimulating agent such as, e.g., a CpG motif or an immune stimulating small nucleotide sequence.

As mentioned above, the adjuvant may comprise one or more cationic substances alone or together with one or more monoglycerides. The total concentration of cationic substances, either alone or if relevant together with one or more monoglycerides in an adjuvant-containing medium is at the most 25% w/v, such as, e.g., at the most 20% w/v, at the most 15% w/v, at the most 10% w/v, at the most 5% w/v, at the most 4% w/v, at the most 3% w/v, at the most 2% w/v or at the most 1% w/v.

A specific embodiment of the invention relates to an adjuvant comprising one or more cationic substances and no monoglycerides, and wherein the total amount of cationic substances in an adjuvant-containing medium is from about 0.1% w/v to about 15% w/v, such as, e.g., from about 0.25% w/v to about 12.5% w/v, from about 0.5% w/v to about 10% w/v, from about 1% w/v to about 7.5% w/v, from about 1% w/v to about 5% w/v, from about 1% w/v to about 4% w/v, from about 1% w/v to about 3% w/v, from about 1% w/v to about 2% w/v or from about 0.5% w/v to about 4% w/v.

The invention also relates to an adjuvant comprising one or more monoglycerides together with one or more cationic substances, wherein the total amount of cationic substances in an adjuvant-containing medium is from bout 0.1% w/v to about 10% w/v, such as, e.g., from about from about 0.25% w/v to about 9% w/v, from about 0.5% w/v to about 8% w/v, from about 1% w/v to about 7% w/v, from about 1% w/v to about 6% w/v, from about 1% w/v to about 5% w/v, from about 1% w/v to about 4% w/v, from about 1% w/v to about 3% w/v, from about 1% w/v to about 2% w/v or from about 0.5% w/v to about 4% w/v.

In one aspect of the invention the medium may further comprise a surface-active agent, which may be hydrophilic and inert and biocompatible, such as, e.g., Pluronic F68.

The medium may further comprise one or more physiologically acceptable additives, such as, e.g., buffering agents, such as, e.g. Tris, stabilizing agents, osmotically active agents, preservatives and pH adjusting agents.

The pH of the medium should be within the physiologically acceptable range, such as from about pH 6 to pH 8. The exact value will be determined based on the pKₐ value of the cationic substances.

An adjuvant according to the present invention can be used for the preparation of a vaccine. Such a vaccine comprises the adjuvant together with an immunogenic quantity of an antigen component and, optionally dispersed in a medium such as an aqueous medium. The vaccine composition may also comprise additional adjuvants, such as, e.g. a monoglyceride as described above.

A specific embodiment of the invention relates to a vaccine comprising one or more cationic substances and no monoglycerides, and wherein the total amount of cationic substances is from about 0.1% w/v to about 15% w/v, such as, e.g., from about 0.25% w/v to about 12.5% w/v, from about 0.5% w/v to about 10% w/v, from about 1% w/v to about 7.5% w/v, from about 1% w/v to about 5% w/v, from about 1% w/v to about 4% w/v, from about 1% w/v to about 3% w/v or from about 1% w/v to about 2% w/v.

The invention also relates to a vaccine comprising one or more monoglycerides together with one or more cationic substances, wherein the total amount of cationic substances is from bout 0.1% w/v to about 10% w/v, such as, e.g., from about from about 0.25% w/v to about 9% w/v, from about 0.5% w/v to about 8% w/v, from about 1% w/v to about 7% w/v, from about 1% w/v to about 6% w/v, from about 1% w/v to about 5% w/v, from about 1% w/v to about 4% w/v, from about 1% w/v to about 3% w/v or from about 1% w/v to about 2% w/v.

For specific non-limiting examples of the composition and preparation of adjuvant and vaccine formulations, please see the enclosed examples.

The antigen component may be selected from the group consisting of antigens from pathogenic and non-pathogenic bacteria, viruses, parasites and tumor cells, or it may be a polynucleotide.

The vaccine formulations comprising an adjuvant according to the invention may be suitable for protection or treatment of animals against a variety of disease states such as, for example, viral, bacterial or parasitic infections, cancer, allergies and autoimmune disorders. Some specific examples of disorders or disease states, which can be protected against or treated by using the methods or compositions according to the present invention, are viral infections caused by hepatitis viruses A, B, C, D & E3 HIV, herpes viruses 1,2, 6 & 7, cytomegalovirus, varicella zoster, papilloma virus, Epstein Barr virus, influenza viruses, para-influenza viruses, adenoviruses, bunya viruses (e.g. hanta virus), coxsakie viruses, picorna viruses, rotaviruses, respiratory syncytial viruses, pox viruses, rhinoviruses, rubella virus, papovavirus, mumps virus and measles virus.

The diseases may also be bacterial infections such as infections caused by Mycobacteria causing TB and leprosy, pneumocci, aerobic gram negative bacilli, mycoplasma, staphyloccocal infections, streptococcal infections, salmonellae and chlamydiae.

The diseases may also be parasitic malaria, leishmaniasis, trypanosomiasis, toxoplasmosis, schistosomiasis, filariasis or various types of cancer such as, e.g. breast cancer, colon cancer, rectal cancer, cancer of the head and neck, renal cancer, malignant melanoma, laryngeal cancer, ovarian cancer, cervical cancer, prostate cancer.

The diseases may also be allergies due to house dust mite, pollen and other environmental allergens and autoimmune diseases such as, e.g. systemic lupus erythematosis

In some embodiments of the invention the adjuvant and/or vaccine formulation is used in a method or composition used to protect against or treat the viral disorders hepatitis B, hepatitis C, human papilloma virus, human immunodeficiency virus, or herpes simplex virus, the bacterial disease TB, cancers of the breast, colon, ovary, cervix, and prostate, or the autoimmune diseases of asthma, rheumatoid arthritis and Alzheimer's. It is to be recognized that these specific disease states have been referred to by way of example only, and are not intended to be limiting upon the scope of the present invention.

Normally, the vaccines may be administered in any convenient manner such as by parental or mucosal administration, such as, e.g. nasal, oral, rectal, vaginal, lung, aural, or skin administration, or by intramuscular, subcutaneous, intradermal or topical routes, and combinations thereof.

The nose is a very attractive route for immunization due to the fact that it is easily accessible, highly vascularized and contains a large absorptions surface. Both mucosal and systemic immune responses can be induced and immune response can be induced at distant mucosal sites, such as the vagina and rectum. Furthermore large populations can easily be immunized, with less risk of infection.

### Legends to figures

**Figure 1** shows responders of IFN gamma secreting cells after intranasal HIV-1 gp160/rev DNA-N3 adjuvant (0-200 µg) immunization pre- and post HIV-1 gp41 peptide booster (N3 adjuvant containing laurylamine and oleylamine). Positive results (values above the mean optical density of the negative control plus two SD) indicate that cytotoxic T-cells are formed.
**Figure 2** shows fecal IgA responses against HIV-1 gp160 envelope antigen pre- and post HIV-1 gp41 peptide booster immunization in HIV-1 gp160/rev DNA-N3 adjuvant (0-200 µg) immunized mice. Fecal wash dilution: 1:4. Positive results (values above the mean optical density or the negative control plus two SD) indicate that a mucosal response has been achieved and that the response has been effected at a distant site, such as in the GI Iract.
**Figure 3** shows serum IgG response against HIV-1 gp160 envelope antigen after intranasal HIV-1 gp160/rev DNA-N3 adjuvant (0-200 µg) immunization pre and post HIV-1 gp41 peptide booster intranasally. Positive results indicate that a systemic immune response has been achieved.
**Figure 4** shows release of IL-2 in mice intranasally HIV-1 gp160/rev DNA-N3 adjuvant (0-200 µg) immunized pre and post HIV-1 gp41 peptide booster. Positive results support the formation of cytoxic T-cells.
**Figure 5** shows release of IL-4 in mice intranasally HIV-1 gp160/rev DNA-N3 adjuvant (0-200 µg) immunized pre and post HIV-1 gp41 peptide booster. Positive results support achievement of a humoral response.

The following examples are intended to illustrate the invention without limiting it in any way.

### EXAMPLES

### Methods

### Preparation of N3 adjuvant and vaccine composition

0.31 g mono-olein and 0.69 g oleylamine was mixed.

To obtain a 4% N3 lipid emulsion was prepared by adding to a beaker 0.4 g of the mixture of mono-olein and oleylamine, 9.6 ml 0.1 M Tris buffer, pH 8.0 and 195 µl 5M HCI. The N3 emulsion was formed by sonication for 2 minutes, whereafter the pH was adjusted to 8.0.

The final vaccine formulation was a 1:1 mixture of the obtained N3 emulsion and a DNA solution with concentration suitable to give the final amounts of DNA used in the Examples (see Table 1).

The different doses of the N3 adjuvant used in the Examples (see Table 1) were obtained by mixing different dilutions of the 4% N3 lipid emulsion described above.

### Immunization

Female 10-12 weeks old C57BI/6 mice of the H-2b haplotype (MTC, Karolinska Institute animal-facility, Stockholm, Sweden) were immunized intranasally with combinations of HIV-1 rgp160BaL DNA, HIV-1 Rev/Lai DNA, gp41/MN coiled coil (aa 578-591, GIKQLQARV- LAVERY) and gp41 subtype A-D peptides (aa 661-675/MN, NEQLLELDKWASLWN, A/92UG31 aa 652-665, EKDLLALDKWANLWN, C/92BR025 aa 651-665, NEQDLL-ALDSWNLWN, D/92UG021 aa 643-657, EQELLKLDQWASLWN) and peptides representing the human 2nd CCR5 coreceptor (aa 168-182, FTRSQKEGLHYTCSSHFPYS) region (Hybaid, T-peptides, Ulm, Germany). The immunization schedule is shown in Table 1.

All DNA plasmids contain the CMV IE promoter for gene expression and the kanamycin gene and were mixed in the N3 adjuvant.

As shown in Table 1 all groups contained 7 mice. The intranasal second booster immunization was performed with a L3/peptide vaccine prepared as follows:

A 8% L3 adjuvant was prepared by combining 44 mg monoglyceride and 36 mg oleic acid in 1 ml of 0.15 M Tris buffer, pH 8.0 followed by sonication and subsequent adjustment of the pH to 8.0. Thereafter a gp41 peptide solution was admixed at a 1:1 ratio to the 8% L3 adjuvant, resulting in a 4% L3/peptide vaccine formulation with a final concentration of the peptide of 8,3 mg/ml.

### Sample collection

Blood was collected by retro-orbital bleedings, fecal pellets were from each mouse. Serum was stored at minus 20°C until used. Fecal pellets were weighted and solubilized in Phosphate Buffered Saline (PBS) pH=7,2-7,4 (0.1 g/ml) with protease inhibitors (1 mg/ml, Sigma-Aldrich). The debris was removed by centrifugation and the supernatant stored at -70°C. When mice were sacrificed intestinal washings were collected.

### Peptide synthesis

Synthetic peptides corresponding to the gp41 neutralizing epitope (aa 661-675/ELDKWAS) (Hybaid T-peptides, Ulm, Germany) representing clade A/(92UG31), B/(MN), C/(92BR25), D/(92UG21); a gp41 clade B/(MN) peptide (Hybaid T-peptides, Ulm, Germany) located between aa 578-595 peptides representing the human and the simian CCR5 N-terminal region and 2nd loop aa 168-182, and aa 178-192 (Hybaid T-peptides, Ulm, Germany, 20, 21), a gp120 V3 loop region clade B aa 302-318 and as negative control a HIV-1 Lai rev-representing peptide aa 65-84 were synthesized by using solid phase F-moc chemistry.

The immunoreactions in the immunized mice were evaluated by the following methods.

### Enzyme-linked immunosorbent assay for detection of IgG and IgA antibodies

Ninety-six well plates (NUNC-Maxisorp, Odense, Denmark) were coated with clade A-D gp41 ELDKWAS peptide, gp41 coil (QLQARVL) peptide, gp120 V3/(MN) (IHIGPGRAFV) and the human CCR5 2nd loop peptides. All peptides were solubilized in 0.1M NaHCO₃ buffer (pH 9.5 to 9.6) at coating concentration of 10 µg/ ml and added at 100 µl/well. Plates were stored overnight at room temperature and at least 24h at 4°C.

Mouse sera were diluted in PBS (pH 7.4) with 0.5% bovine serum albumine (BSA, Boehring Mannheim, Mannheim, Germany) and 0.05% Tween 20 (Sigma, Aldrich, Sweden, AB) and 100 µl of dilutions 1:50, 1:250, 1:1250 and 1:6250 were added to each well, and incubated for 90 min at 37°C. Mucosal samples were tested as follows. Plates were blocked with 5% Blotto for 1 h at 37°C and mucosal samples were diluted 1/2 - 1/56 in two-fold dilutions in 2.5% Blotto and added 100 µl/well. Plates were incubated at 4°C for 16 h and washed. Bound antibodies were detected with anti-murine IgA- and IgG specific conjugates: Horseradish peroxidase conjugated anti-mouse IgG (Biorad, Richmond), dilution 1:2000 or anti-mouse IgA (Southern Biotechnologies, Birmingham, AL) dilution 1:1000, was added at 100 µl/well, incubated for 2 h at 37°C, and OPD-reagent (2 mg/ml orthophenylendiamine in 0.05 M sodium citric acid pH 5.5 with 0.003% H₂O₂) was added as substrate at 100 µl/well. After a 30 minutes incubation period, the reaction was stopped by adding 100 µl/well 2.5M H₂SO₄. Absorbance was measured at 490nm. Values above the mean optical density of the negative control plus two SD were used as the cut off and values above were considered as positive.

To determine the specific titer of antibodies directed to gp160, 96-well plates (NUNC-Maxisorp, Odense, Denmark) were coated with a recombinant gp160. This protein was solubilized in 0.1M NaCO₃ buffer (pH 9.5 to 9.6) at coating concentration of 1 µg/ml. Plates were stored over night at room temperature and for a minimum of 24 h at 4°C. Patient sera was diluted in ten fold dilutions starting with 1:100 in PBS (pH 7.4) with 0.5% bovine serum albumine (BSA, Boehring Mannheim, Mannheim, Germany) and 0.05% Tween 20 (Sigma, Aldrich, Sweden, AB). After washing sera were diluted 1:100, 1:1000, 1:10000 and 1:100000 and 100 µl/well were added and incubated for 90 min at 37°C. Horse peroxidase conjugate anti-human IgG (Biorad, Richmond) in dilution 1:40000 were added for 2 h at 37°C. After washing, 100 µl/well OPD (2 mg/ml orthophenylendiamine in 0.01 M sodium citric/citric acid pH 5.5 with 0.03% H₂O₂) was added as substrate and the reaction mixture was incubated for 30 min. Reaction was stopped by adding 2.5M H₂SO₄. The absorbance was measured at 490nm. Values above the mean optical density of the negative control plus 2 SD were considered as positive.

The positive control used was a human HIV-lgG pool collected from HIV-1 infected Ugandan patients, the Kabi 62 serum, monoclonal antibodies against the gp41 ELDKWAS epitope Mab 2F5 (donated by Dr.H. Katinger), monoclonal anti-gp120 V3 antibody F58/H3 and monoclonal antibodies against the 2nd CCR5 external loop Mab 2D7 (Coulter Pharmaceuticals, Palo Alto, CA). As negative controls pre-immunization serum, pre-immunization vaginal washings and fecal pellets were used. Quantification of IgA and IgG was performed by capture ELISA with commercially available IgA (1 mg/ml) and IgG (2 mg/ml) standards (Sigma). Values above the mean optical density of the negative control plus two SD were used as the cut off and values above were considered as positive.

### IgA purification and quantitation

The fluids collected from the intestinal/fecal washings were used to isolate and analyse the IgA content. The Kaptive IgA/IgE reagents (Biotech IgG, Copenhagen, Denmark) were purchased and used as recommended by the manufacturer. Reagents for preparing an in-house murine IgA capture ELISA were purchased and a commercial murine IgA (1 mg/ml, Sigma-Aldrich) was used for preparing a standard curve. The purified IgA and the standard IgA was diluted in PBS (pH=7.2-7.4) with 5% fat-free dry-milk, 0.05% Tween 20 at ten-fold serial dilutions. One hundred micro liters per dilution was added to 96-microwellplate wells precoated with rabbit anti-murine IgA (Dakopatts AB, Sollentuna, Sweden) and incubated 1 h at 37°C. The plate wells were washed 4 times with saline with 0.05% Tween 20 before 100 µl HRP-conjugated goat anti-murine IgA was added to each well. After 1 h incubation at 37°C plates were washed as previously described and the presence of bound conjugate was detected by using o-phenylene diamine in 0.05M sodium-citric acid, activated with 0.03% H₂O₂ OPD-reagent as substrate. The substrate reaction was terminated with 100 µl/well 2.5M H₂SO₄ and the absorbance was measured at OD490. The amounts of IgA in the mouse-samples were determined by comparing the OD-values of the test samples with the IgA standard.

### B-cell memory, IgG/IgA synthesis in vitro

1x10⁵ spleen cells were cultured in 200 µl of RPMI 1640 medium (Life Technologies, Scotland, UK) supplemented with 5% inactivated fetal calf serum at 37°C in 96-flat bottomed cell culture plates (NUNC) with rgp160 (1 µg/ml) or with peptides (10 µg/ml) for 72 hrs. After washing, antigen-specific immunoglobulins were measured by ELISA as mentioned above. A positive reactivity was considered if the ELISA substrate absorbance was higher than the mean OD+2SD of the negative control mice.

Lymph nodes were collected from sacrificed mice. The lymph nodes cells were pooled for each group of mice due to low total numbers of available cells from each individual mouse. 1x10⁵ lymph node cells were cultured in 200 µl of RPMI 1640 supplemented with 5% inactivated fetal calf serum at 37°C in 96-flat bottomed cell culture plates with rgp160 (1µg/ml) or with peptides (10 µg/ml) for 72 hrs. After washing, antigen-specific immunoglobulins were measured by ELISA as mentioned above. A positive reactivity was considered if the ELISA substrate absorbance was higher than the mean OD+2SD of the negative control mice.

### N3/L3 adjuvant for HIV-1 gp160/rev DNA and gp41 peptide booster immunization

### Immunization schedule

3 days prior to the start of the study: Pre-bleed of mice.

WEEK 0. Primary immunization, each group contains 7 mice and each was immunized intranasally with 10 µl of the vaccine formulations. By using only 10 µl of the vaccine formulation it is ensured that the vaccine formulation is only applied to the nose of the mice, and not to the mouth and lungs.

**Table 1**

| **Group** | **µg of gp160-Rev** | **Dose N3 (µg)^{*}** |
|---|---|---|
| **1** | **8** | **200** |
| **2** | **8** | **100** |
| **3** | **8** | **50** |
| **4** | **8** | **20** |
| **5** | **8** | **0** |
| **6** | **0,8** | **100** |
| **7** | **0,8** | **0** |

| | | |
|---|---|---|
| total amount of mono-olein and oleyl amine | | |

WEEK 4: Blood sampling from mice for immunoanalyses.

WEEK 7: Booster-immunization with 10 µg gp41 peptide in 4% L3-adjuvant intranasally (6 µl/nostril) to all mice in groups 1-6.

Mice in group 7 received the gp41 peptides diluted in saline (10 µg/mouse, 6 µl/nostril).

WEEK 10: Blood sampling and immunoassays.

WEEK 12: Mice sacrificed. Blood and spleen cell analyses.

### Results

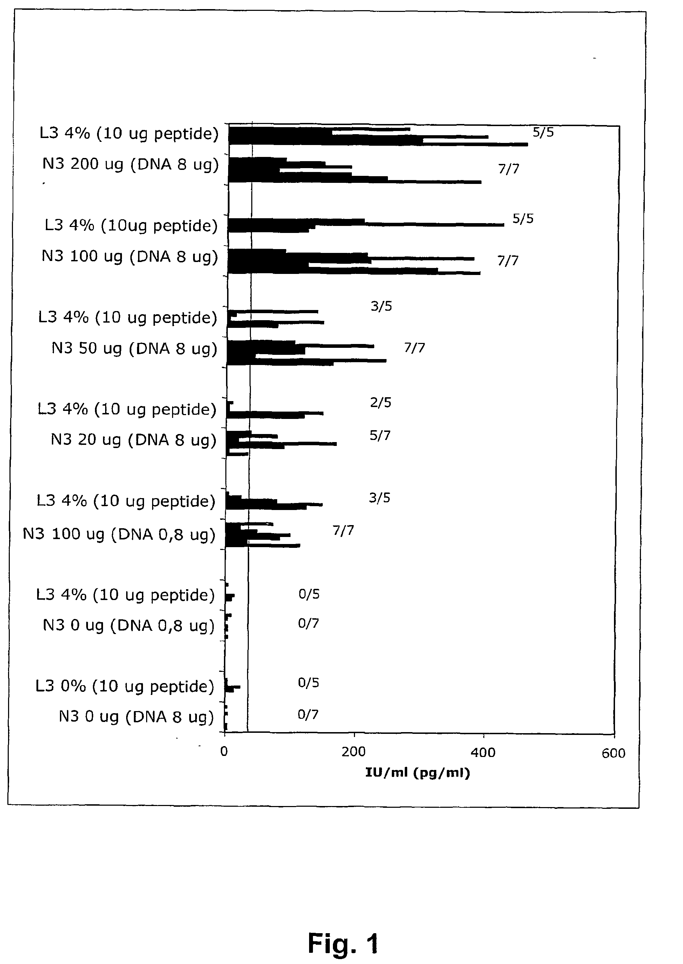

The results from the study are shown in Figures 1-5.

The results are seen as bars with two sets of data for each group of mice. The first (lower datagroup) shows the results obtained 4 weeks after the DNA administration and the second shows the immune response three weeks after the booster vaccination. All results indicate that unless the DNA is co-administered with the N3 adjuvant, no immune response above cut-off level can be detected in any of the groups when only 10 µl vaccine formulation is used for the nasal administration. Also shown is the dose-dependent response of the N3 adjuvant according to the present invention.

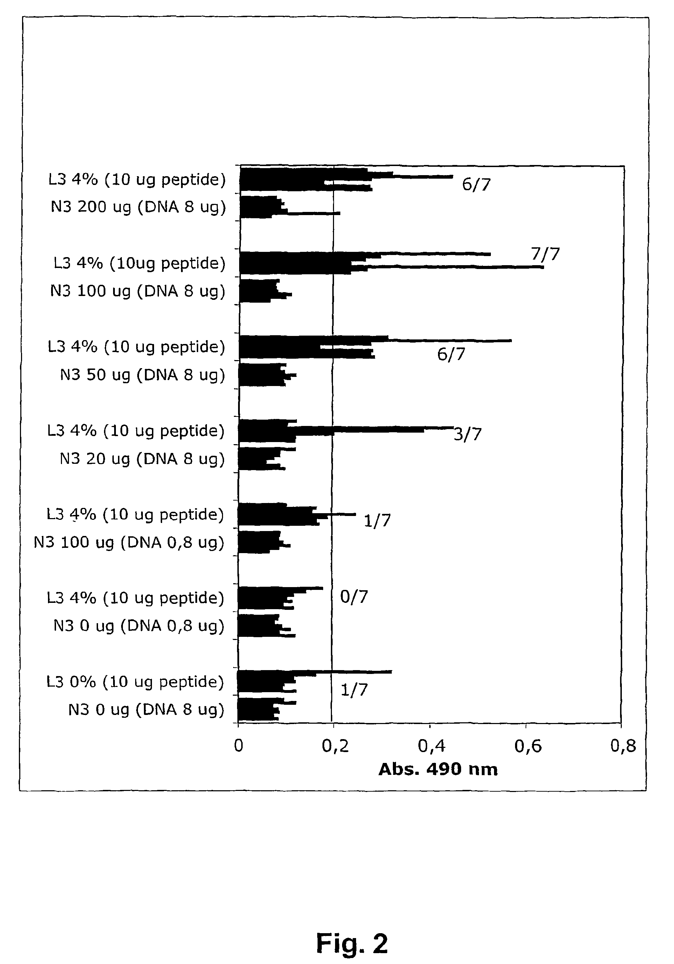

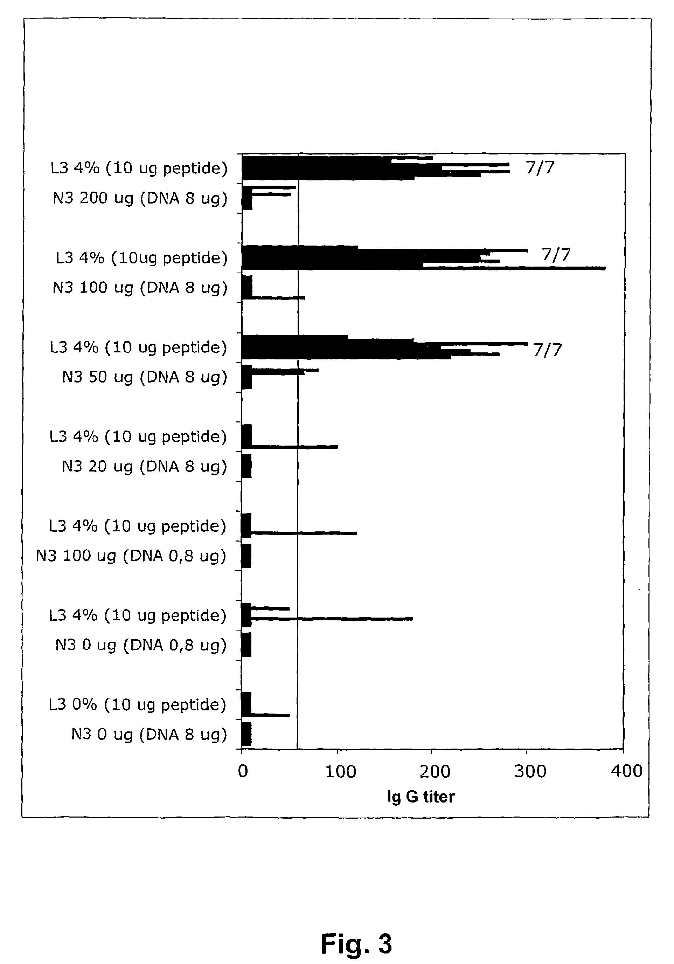

With respect to the immune responses obtained after the first immunization with HIV-1 gp160 DNA alone, no or only few or weak antibody responses were seen in serum (Figure 3) and intestinal samples (Figure 2). No difference was seen in animals receiving DNA with or without the N3 adjuvant.

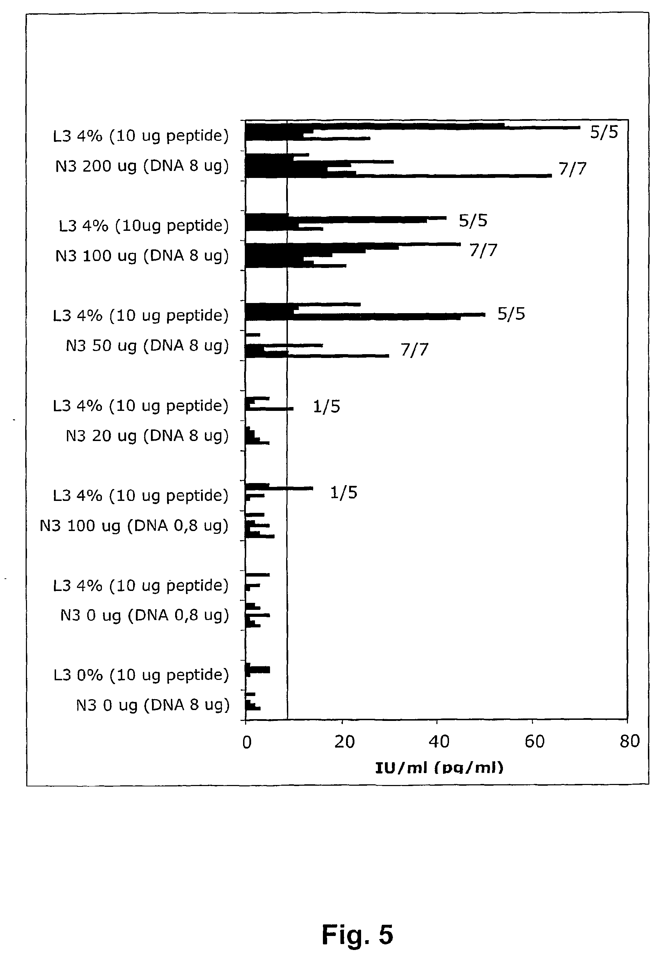

As for the cell-mediated immunity (Figure 1) almost all (6/7 - 7/7) animals receiving 8 µg DNA with 50 µg of N3 or more responded by developing IFN-gamma releasing T-cells suggesting that a cytotoxic T cell response was evoked against the HIV-1 envelope antigen. In animals receiving lower N3 adjuvant concentrations or lower gp160-DNA amounts than 8 µg, between 2-4/7 animals responded by INF-gamma responses. No HIV-1 envelope specific responses were seen in animals receiving DNA without the N3 adjuvant. Another marker for cell-mediated immunity is IL-2. As can be seen in Figure 5, the same pattern is seen as for IFN-gamma, further strengthening the suggestion that a cytotoxic T cell response has been evoked.

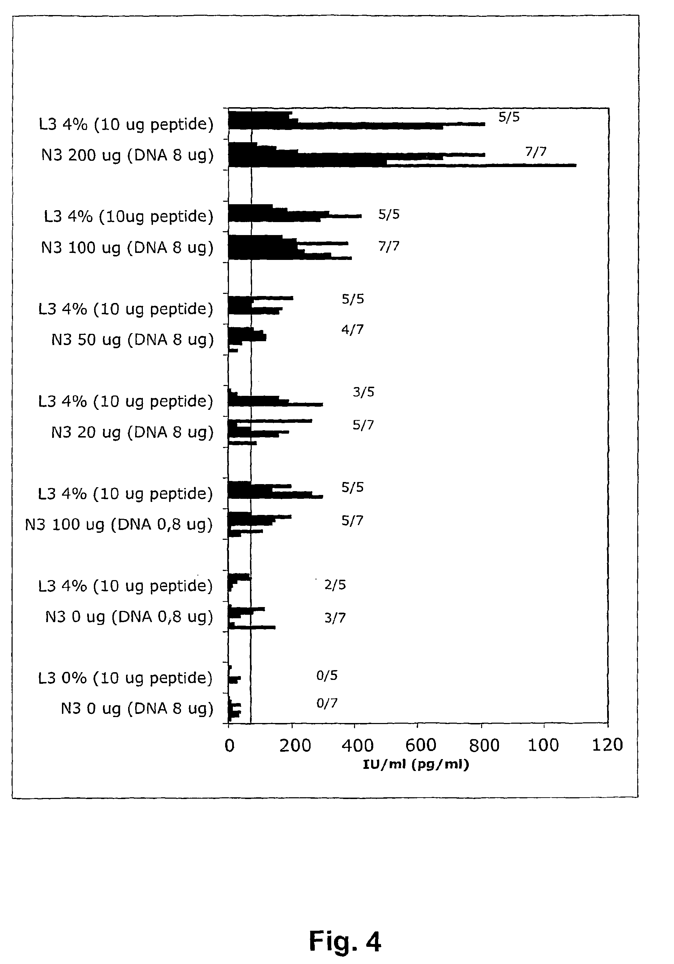

With respect to the immune responses after the HIV-1 gp41/L3 peptide booster immunization (Figure 2 and 3), practically all animals immunized with 8 µg gp160-DNA/N3 followed by gp41 peptide in 4% L3 adjuvant developed serum IgG and intestinal IgA specific for HIV-1 envelope antigen and the HIV-1 gp41 transmembrane protein. Poor antibody responses were seen in animals receiving gp41 peptides without the L3 adjuvant (1/7 developed low amounts of antibodies to HIV-1 envelope). Another marker for a humoral immune response is IL-4. As can be seen in Figure 4, a response of IL-4 can be seen, further strengthening the suggestion that a humoral immune response has been evoked.

As for the cell-mediated immunity all animals receiving 8 µg DNA with 100 µg of N3 or more and gp41 peptide in 4% L3 adjuvant responded by developing IFN-gamma releasing T-cells suggesting that a cytotoxic T cell response was evoked against the HIV-1 envelope antigen. In animals receiving lower N3 adjuvant concentrations or lower gp160-DNA amounts than 8 µg, between 3-4/5 animals responded by INF-gamma responses. Animals immunized without the N3 adjuvant did not respond by developing detectable cell-mediated immunity.

Furthermore, when DNA was administered together with L3 instead of the N3 adjuvant, the cell-mediated immunity induced were the same as for DNA without adjuvant (in distilled water or saline), indicating that DNA and L3 is not an immune enhancing combination.

### Conclusions

A clear N3-adjuvant dose-dependent enhancing effect both on antibody reactivity in serum and on mucosal surfaces was seen. At least a dose of 50 µg N3 adjuvant emulsified in a medium was needed to obtain a high frequency of IgG, IgA and IL-4 immune response.

A clear N3-adjuvant dose-dependent effect was also seen on the cell-mediated immune responses towards the HIV-1 envelope antigen. The highest amounts of INF-gamma and IL-2 cytokine responses were seen in animals obtaining a dose of 100 µg adjuvant or more, suggesting that these animals developed higher amounts of cytotoxic T cells against the HIV-1 envelope.

## Claims

1. A composition comprising one or more acyl amines comprising from 4 to 30 carbon atoms for use as an adjuvant in a vaccine.

2. A composition according to claim 1, which - as a further adjuvant component - comprises a monoglyceride with a purity of at least 80% w/w, the monoglyceride having the formula 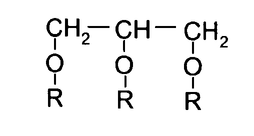 wherein R is selected from H and an acyl group containing from 6 to 30 carbon atoms with the proviso that two of the R groups are H.

3. A composition according to any of the preceding claims wherein the adjuvant components, i.e. the acyl amines and optionally monoglycerides are present in a concentration that elicits an immune response when administered to an animal.

4. A composition according to any of the preceding claims wherein the acylamines are saturated or unsaturated.

5. A composition according to any of the preceding claims, wherein the acyl amines are selected from the group consisting of lauryl amine (C12), palmityl amine (C16), palmitoleyl amine (C16:1), oleyl amine (C18:1) and linoleyl amine (C18:2).

6. A composition according to any of the preceding claims comprising a mixture of oleyl amine and lauryl amine.

7. A composition according to any of the preceding claims further comprising an aqueous medium.

8. A composition according to claim 7, wherein the aqueous medium comprises an immune stimulating substance, selected from CpG motifs.

9. A composition according to claims 7-8, wherein the aqueous medium further comprises the surface-active agent Pluronic F68.

10. A composition according to any of claims 7-9, wherein the medium further comprises one or more physiologically acceptable additives selected from buffering agents, stabilising agents, osmotically active agents, preservatives and pH adjusting agents.

11. A composition according to any of claims 8-12, wherein the total concentration of cationic substances, either alone or if relevant together with monoglycerides, in the medium is at the most 25% w/v, or at the most 20% w/v, at the most 15% w/v, at the most 10% w/v, at the most 5% w/v, at the most 4% w/v, or at the most 3% w/v.

12. A vaccine composition comprising an adjuvant according to any of the preceding claims together with an antigen component.

13. A vaccine composition according to claim 12, wherein the antigen component is selected from the group consisting of antigens from pathogenic and non-pathogenic bacteria, viruses, parasites and tumor cells.

14. A vaccine composition according to claim 13, wherein the antigen component is a polynucleotide.

15. A vaccine composition according to any of claims 12-14 further containing an aqueous medium.

16. A vaccine composition according to any of claims 12-15, wherein the adjuvant comprises a mixture of mono-olein and oleyl amine.

17. A vaccine composition according to claim 16, wherein the w/w ratio of mono-olein and oleyl amine is 0.45.

18. A vaccine composition according to claim 16 or 17, wherein the total amount of mono-olein and oleyl amine is at least 40 µg, or at least 50 µg, or at least 55 µg, or at least 60 µg, or at least 70 µg, or at least 80 µg, or at least 90 µg or at least 100 µg.

19. A vaccine composition according to any of claims 12-18, wherein the composition comprises additional adjuvants.

20. A vaccine composition according to any of claims 12-19, wherein the composition is in a form suitable for parenteral or mucosal administration.

21. A vaccine composition according to claim 20, wherein the composition is in a form suitable for administration to the mucosa of the nose, mouth, vagina, rectum or intestine.

## Patentansprüche

1. Zusammensetzung, die ein oder mehrere Acylamine umfasst, die 4 bis 30 Kohlenstoffatome umfassen, zur Verwendung als ein Adjuvans in einem Impfstoff.

2. Zusammensetzung nach Anspruch 1, die - als eine weitere Adjuvanskomponente - ein Monoglycerid mit einer Reinheit von mindestens 80 Gew.-% umfasst, worin das Monoglycerid die Formel aufweist 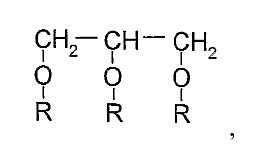 worin R ausgewählt ist unter H und einer Acylgruppe, die 6 bis 30 Kohlenstoffatome umfasst, ausgenommen, dass zwei der R Gruppen H sind.

3. Zusammensetzung nach einem der vorstehenden Ansprüche, worin die Adjuvanskomponenten, das heißt die Acylamine und wahlweisen Monoglyceride in einer Konzentration vorliegen, die, wenn sie einem Tier verabreicht wird, eine Immunantwort hervorruft.

4. Zusammensetzung nach einem der vorstehenden Ansprüche, worin die Acylamine gesättigt oder ungesättigt sind.

5. Zusammensetzung nach einem der vorstehenden Ansprüche, worin die Acylamine ausgewählt sind aus der Gruppe bestehend aus Laurylamin (C12), Palmitylamin (C16), Palmitoleylamin (C16:1), Oleylamin (C18:1) und Linoleylamin (C18:2).

6. Zusammensetzung nach einem der vorstehenden Ansprüche, die ein Gemisch von Oleylamin und Laurylamin umfasst.

7. Zusammensetzung nach einem der vorstehenden Ansprüche, die weiterhin ein wässriges Medium umfasst.

8. Zusammensetzung nach Anspruch 7, worin das wässrige Medium eine immunstimulatorische Substanz ausgewählt aus der Gruppe bestehend aus CpG Motiven umfasst.

9. Zusammensetzung nach einem der Ansprüche 7-8, worin das wässrige Medium weiterhin das oberflächenaktive Mittel Pluronic F68 umfasst.

10. Zusammensetzung nach einem der Ansprüche 7-9, worin das Medium weiterhin einen oder mehrere physiologisch verträgliche Hilfsstoffe ausgewählt aus der Gruppe bestehend aus Puffermitteln, stabilisierenden Mitteln, osmotisch aktiven Mitteln, Konservierungsmitteln und pH einstellenden Mitteln umfasst.

11. Zusammensetzung nach einem der Ansprüche 8-10, worin die Gesamtkonzentration kationischer Substanzen, entweder alleine oder falls relevant zusammen mit Monoglyceriden, in dem Medium höchstens 25 % Gew./Vol., oder höchstens 20 % Gew./Vol., höchstens 15 % Gew./Vol., höchstens 10 % Gew./Vol., höchstens 5 % Gew./Vol., höchstens 4 % Gew./Vol., oder höchstens 3 % Gew./Vol. beträgt.

12. Impfstoffzusammensetzung umfassend ein Adjuvans nach einem der vorstehenden Ansprüche zusammen mit einer Antigenkomponente.

13. Impfstoffzusammensetzung nach Anspruch 12, worin die Antigenkomponente ausgewählt ist aus der Gruppe bestehend aus Antigenen von pathogenen und nicht pathogenen Bakterien, Viren, Parasiten und Tumorzellen.

14. Impfstoffzusammensetzung nach Anspruch 13, worin die Antigenkomponente ein Polynukleotid ist.

15. Impfstoffzusammensetzung nach einem der Ansprüche 12-14, das weiterhin ein wässriges Medium umfasst.

16. Impfstoffzusammensetzung nach einem der Ansprüche 12-15, worin das Adjuvans ein Gemisch aus Monoolein und Oleylamin umfasst.

17. Impfstoffzusammensetzung nach Anspruch 16, worin das Gew./Gew.-Verhältnis von Monoolein und Oleylamin 0,45 beträgt.

18. Impfstoffzusammensetzung nach einem der Ansprüche 16 oder 17, worin die Gesamtmenge von Monoolein und Oleylamin mindestens 40 µg, oder mindestens 50 µg, oder mindestens 60 µg, oder mindestens 70 µg, oder mindestens 80 µg, oder mindestens 90 µg oder mindestens 100 µg beträgt.

19. Impfstoffzusammensetzung nach einem der Ansprüche 12-18, worin die Zusammensetzung zusätzliche Adjuvantien umfasst.

20. Impfstoffzusammensetzung nach einem der Ansprüche 12-19, worin die Zusammensetzung in einer Form vorliegt, die für eine parenterale oder mukosale Verabreichung geeignet ist.

21. Impfstoffzusammensetzung nach Anspruch 20, worin die Zusammensetzung in einer Form vorliegt, die geeignet ist der Mucosa der Nase, des Mundes, der Vagina, des Rektums oder des Darms verabreicht zu werden.

## Revendications

1. Une composition comprenant une ou plusieurs amines acylées comprenant de 4 à 30 atomes de carbone pour une utilisation comme un adjuvant dans un vaccin.

2. Une composition selon la revendication 1, qui - en tant que composant adjuvant supplémentaire - comprend un monoglycéride avec une pureté d'au moins 80% pds/pds, le monoglycéride ayant pour formule: 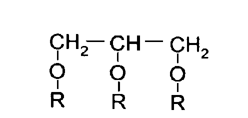 dans laquelle R est choisi parmi H et un groupe acyl contenant de 6 à 30 atomes de carbone à la condition que deux des groupes R soient H.

3. Une composition selon l'une quelconque des revendications précédentes, dans laquelle les composants adjuvant, à savoir les amines acylées et éventuellement les monoglycérides sont présents à une concentration qui suscite une réponse immune lorsqu'ils sont administrés à un animal.

4. Une composition selon l'une quelconque des revendications précédentes, dans laquelle les amines acylées sont saturées ou insaturées.

5. Une composition selon l'une quelconque des revendications précédentes, dans laquelle les amines acylées sont choisies parmi le groupe consistant en de l'amine lauryle (C12), amine palmityle (C16), amine palmitoleyle (C16:1), amine oleyle (C18:1) et amine linoleyle (C18:2).

6. Une composition selon l'une quelconque des revendications précédentes, comprenant un mélange d'amine oleyle et d'amine lauryle.

7. Une composition selon l'une quelconque des revendications précédentes, comprenant en outre un milieu aqueux.

8. Une composition selon la revendication 7, dans laquelle le milieu aqueux comprend une substance stimulant le système immun, choisie parmi des motifs CpG.

9. Une composition selon les revendications 7-8, dans laquelle le milieu aqueux comprend en outre l'agent actif de surface Pluronic F68.

10. Une composition selon l'une quelconque des revendications 7-9, dans laquelle le milieu comprend en outre un ou plusieurs additifs physiologiquement acceptables choisis parmi des agents tampons, des agents stabilisants, des agents osmotiquement actifs, des conservateurs et des agents modificateurs de pH.

11. Une composition selon l'une quelconque des revendications 8-12, dans laquelle la concentration totale de substances cationiques, soit seule soit le cas échéant en association avec des monoglycérides, dans le milieu est au plus de 25% pds/v, ou au plus de 20% pds/v, au plus de 15% pds/v, au plus de 10% pds/v, au plus de 5% pds/v, au plus de 4% pds/v, ou au plus de 3% pds/v.

12. Une composition vaccinale comprenant un adjuvant selon l'une quelconque des revendications précédentes, en association avec un composant antigène.

13. Une composition vaccinale selon la revendication 12, dans laquelle le composant antigène est choisi parmi le groupe consistant en des antigènes de bactéries pathogènes et non-pathogènes, des virus, des parasites et des cellules tumorales.

14. Une composition vaccinale selon la revendication 13, dans laquelle le composant antigène est un polynucléotide.

15. Une composition vaccinale selon l'une quelconque des revendications 12-14 contenant en outre un milieu aqueux.

16. Une composition vaccinale selon l'une quelconque des revendications 12-15, dans laquelle l'adjuvant comprend un mélange d'amine mono-oléine et oleyle.

17. Une composition vaccinale selon la revendication 16, dans laquelle le rapport pds/pds d'amine mono-oléine et oleyle est de 0,45.

18. Une composition vaccinale selon la revendication 16 ou 17, dans laquelle la quantité totale d'amine mono-oléine et oleyle est au moins de 40 µg, ou au moins de 50 µg, ou au moins de 55 µg, ou au moins de 60 µg, ou au moins de 70 µg, ou au moins de 80 µg, ou au moins de 90 µg ou au moins de 100 µg.

19. Une composition vaccinale selon l'une quelconque des revendications 12-18, dans laquelle la composition comprend des adjuvants supplémentaires.

20. Une composition vaccinale selon l'une quelconque des revendications 12-19, dans laquelle la composition est sous une forme appropriée pour une administration parentérale ou par les muqueuses.

21. Une composition vaccinale selon la revendication 20, dans laquelle la composition est sous une forme appropriée pour une administration aux muqueuses du nez, de la bouche, du vagin, du rectum ou de l'intestin.
